# EUROPEAN PATENT SPECIFICATION

(11) **EP 1 480 677 B1**
(45) Date of publication and mention of the grant of the patent: **29.02.2012**
(21) Application number: 02795068.2
(22) Date of filing: 20.11.2002
(51) Int. Cl.: A61K 47/06, A61K 47/36, A61K 9/08, A61P 27/02

(54) **COMPOSITION FOR STABILIZING HYALURONIC ACID**
ZUSAMMENSETZUNG ZUR STABILISIERUNG VON HYALURONSÄURE
COMPOSITION DE STABILISATION DE L'ACIDE HYALURONIQUE

(30) Priority: 21.11.2001 US 332042 P
(43) Date of publication of application: 01.12.2004
(73) Proprietor: IOLTECH, 17180 Perigny (FR); Novartis Pharma GmbH, 1230 Wien (AT)
(72) Inventor: TSAO, Fu-Pao, Lawrenceville, GA 30043 (US)
(74) Representative: Lewitter, Herbert
(86) International application number: PCT/EP2002/013019
(87) International publication number: WO 2003/043660

(56) References cited:
- EP-A- 0 938 896
- US-A- 5 858 996
- GOA K L ET AL: "HYALURONIC ACID A REVIEW OF ITS PHARMACOLOGY AND USE AS A SURGICAL AID IN OPHTHALMOLOGY, AND ITS THERAPEUTIC POTENTIAL IN JOINT DISEASE AND WOUND HEALING" DRUGS, ADIS INTERNATIONAL LTD, AT, vol. 47, no. 3, 1994, pages 536-566, XP009007356 ISSN: 0012-6667

## Description

The present invention provides compositions and methods for reducing the decomposition rate of hyaluronic acid at concentrations above 0.3%. The compositions include at least one strong, stable chelating agent, preferably an organophosphorous compound such as diethylene triamine penta(methylene phosphonic acid). These biocompatible compositions are especially useful in the ophthalmic field.

### BACKGROUND OF THE INVENTION

### Field of the Invention

This invention relates broadly to compositions and method for stabilizing hyaluronic acid or the salts thereof at concentrations above 0.3%. In a preferred embodiment, the invention relates to stabilization of sodium hyaluronate in ophthalmic compositions.

### Description of the Related Art

Poly (carboxylic acids) and the salts thereof are known to be useful in eye drops for managing dry eye syndrome. For example, hyaluronic acid is used in ophthalmic solutions or mixtures for this purpose. An example of a commercially available sodium hyaluronate is BS5111 available from Fermentech.

Typically, poly (carboxylic acids) such as sodium hyaluronate decompose, or are otherwise altered, during extended storage periods. For example, as hyaluronate is degraded, the distribution of molecular weight of the polymer decreases. The decomposition of this ingredient reduces the effectiveness of the composition, eventually to a point at which the composition is no longer deemed sufficiently effective for its intended use. Thus, there exist shelf-life problems with compositions, most notably buffered ophthalmic compositions that include poly (carboxylic acids). Accordingly, there is a need to reduce the rate of decomposition of poly (carboxylic acids), and to increase the shelf life of compositions including these materials.

U.S. Patent No. 5,576,028 to Martin, et al*.* teaches reducing the decomposition rate of hydrogen peroxide. The compositions include at least one strong, stable chelating agent, preferably an organophosphorous compound such as diethylene triamine penta(methylene phosphonic acid). These biocompatible compositions are disclosed as being especially useful in the ophthalmic field. While stabilized hydrogen peroxide solutions containing a poly (carboxylic acids) at concentrations much less than 0.3% are disclosed, there is no teaching or suggestion of poly (carboxylic acids) at 0.3% or greater nor that such poly (carboxylic acids) are stabilized.

U.S. Patent No. 5,858,996 to Tsao teaches reducing the decomposition rate of viscosity enhancers, such as poly(acrylic acids) - but not poly (carboxylic acids). The compositions include at least one strong, stable chelating agent, preferably an organophosphorous compound such as diethylene triamine penta(methylene phosphonic acid). These biocompatible compositions are disclosed as being especially useful in the ophthalmic field.

Three commercially available hyaluronates for use in ophthalmic surgery are as follows:
a. HEALON® - each ml of HEALON contains 10 mg of sodium hyaluronate, 8.5 mg of sodium chloride, 0.28 mg of disodium dihydrogen phosphate dihydrate, 0.04 mg of sodium dihydrogen phosphate hydrate and q.s. water for injection USP.
b. AMVISC® - each ml of AMVISC contains 10 mg of sodium hyaluronate adjusted to yield approximately 40,000 centistokes, 9.0 mg of sodium chloride and sterile water for injection USPQS.
c. VISCOAT® - each 1 ml of VISCOAT solution contains not more than 40 mg of sodium chondroitin sulfate, 30 mg sodium hyaluronate, 0.45 mg sodium dihydrogen phosphate hydrate, 2.00 mg disodium hydrogen phosphate, 4.3 mg sodium chloride (with water for injection USP grade, qs).

None of these three products contains a stabilizer, in particular a strong chelating agent. Without these stabilizers, there is the potential for degradation of the hyaluronic acid, and the subsequent loss of protective efficacy.

### SUMMARY OF THE INVENTION

One embodiment of the invention is a stabilized buffered composition, which includes at least hyaluronic acid or salt thereof at a concentration greater than 0.3%, and at least one strong chelating agent (e.g., a phosphonic acid-containing chelating agent) capable of complexing with trace amounts of free catalytic metal ions. The chelating agent is believed to complex with trace amounts of metal ions, thereby reducing the free metal ion concentration. This reduction in free metal ion concentration reduces the decomposition rate of the poly (carboxylic acid). The compositions, which are especially useful in the ophthalmic field, exhibit increased shelf life.

Accordingly, in one aspect, the present invention relates to a stabilized solution, comprising:
(a) greater than about 0.3 weight percent of at least hyaluronic acid and salts thereof and mixtures thereof; and
(b) at least one strong and stable chelating agent having at least one phosphonic acid group in an amount of from 0.0001 to 0.1 weight percent; and
(c) water.

Another embodiment of the invention is a method of stabilizing hyaluronic acid at a concentration greater than 0.3%. The method involves providing an ophthalmically compatible composition including hyaluronic acid, adding a strong (e.g., a phosphonic acid-containing) chelating agent to the composition, and allowing the chelating agent to complex with free catalytic metal ions in the composition. The composition exhibits a decomposition rate that is less than the decomposition rate of a composition that does not include a strong chelating agent. Therefore, the resultant composition has an improved shelf life.

Thus, in another aspect, the present invention relates to a method of stabilizing a hyaluronic acid composition, comprising the step of preparing said hyaluronic acid composition including about 1 to 10 weight percent of hyaluronic acid or salts or mixture thereof and at least one strong and stable amino tri(lower alkylene phosphonic acid) chelating agent in an amount of from 0.0001 to 0.1 weight percent, wherein said chelating agent is capable of complexing with free catalytic metal ions to produce a composition with metal ion complexes and whereby reducing the decomposition rate of the hyaluronic acid in said composition. Yet a further embodiment of the present invention is a composition having a free metal ion concentration less than an amount that will cause substantial hyaluronic acid decomposition over a one-year storage period at room temperature. Also described herein is a method of performing surgery on an eye including employing the hyaluronic acid composition of the present invention during the performance of said surgery.

### DESCRIPTION OF THE PREFERRED EMBODIMENTS

The solutions of the present invention include hyaluronic acid at a concentration greater than 0.3%, a buffer, and a stabilizer. Preferably, the concentration of the hyaluronic acid is greater than 0.8%. A preferred group of solutions are those which are ophthalmically acceptable, i.e., those which do not produce substantial irritation or damage when contacted with the eye, ocular tissue, or surrounding fluids. The preferred ophthalmic solutions are those that are aqueous.

"Molecular weight" of a polymeric material, as used herein, refers to the number-average molecular weight unless otherwise specifically noted or unless testing conditions indicate otherwise.

Preferred stabilizers of the present invention are a group of chelating agents having phosphonic acid or phosphonate groups. A preferred group of chelating agents is organophosphonates, particularly amino tri (lower alkylene phosphonic acids). A variety of such chelating agents are commercially available from Monsanto Company, St. Louis, Mo., and are sold under the trademark DEQUEST®. Examples of such compounds include, without limitation, diethylene triamine penta(methylene phosphonic acid); hexamethylenediaminetetra (methylenephosphonic acid); ethylenediaminetetra (methylenephosphonic acid); and aminotrimethylene phosphonates. A particularly preferred chelating agent is diethylene triamine penta(methylene phosphonic acid), sold under the trademark DEQUEST® 2060.

The poly (carboxylic acids) is preferably selected from the group consisting of hyaluronic acids, preferably the salts thereof, and most preferably sodium hyaluronate.

Hyaluronic acid (HA) is a typical and important representative of a class of biological macromolecules known as glycosaminoglycans (mucopoly-saccharides). HA is a biological polymer that is present, with identical molecular structure, in all connective tissues of vertebrate organisms, where it plays a structural and biological role, in the sense that its local levels are strictly correlated with the tonus, trophism and tissue repair in case of injury. A review on the physiological role of these biological substances was given in Phys. Rev. (Comper, Laurent: Physiological Function Of Connective Tissue Polysaccharides, Phys. Rev., 58, (1), 255-315, 1978). The chemical-physical nature of HA is that of a saccharide biopolymer (D-glucuronic acid and N-acetylglycosamine), polymerized in alternation, forming long, unbranched molecular chains varying in molecular weight to a maximum of 8,000,000 Daltons (Meyer; Chemical Structure of Hyaluronic Acid. Fed. Proceed. 17, 1075, 1958; Laurent; Chemistry and Molecular Biology of Intracellular Matrix, 703-732, Academic Press N. Y., 1970). The behaviour of this biopolymer in aqueous solution guarantees a particular viscosity, called viscoelasticity, which is typical of some biological fluids, such as synovial fluid and vitreous fluid, where HA is present at a concentration of 0.12 - 0.24% (Balzas, et al.: Hyaluronic acid and replacement of vitreous and aqueous humor. Mod. Probl. Ophthal., 10, 3-21, 1972). Also aqueous humor, of human origin, was found to contain HA in an average concentration of 1.14 mg/g (Laurent: Hyaluronate In Human Aqueous Humor, Arch. Ophthalmol., 101, 129-130,1983).

A body of published evidence has accumulated showing that the local supply of exogenous HA has distinct therapeutic and protective benefits in a great variety of pathological conditions of connective and epithelial tissues, such as:
- impaired tissue regeneration in non-healing skin ulcers;
- arthrosic degeneration of articular connective tissue;
- ocular surgery.

Particularly appreciated is the possibility, provided by the visco-elastic nature of HA, to coat the tissues exposed to risk of damage during surgical manipulation. According to all the surgeons who have used HA, the presence of a viscous layer of exogenous HA on the tissues which are most exposed to traumatizing accidental contacts, such as the cornea, exerts an efficient protective influence, which is reflected to a very positive degree in the successful outcome of the operation.

The protective effect and the facilitatory influence on tissue repair exerted by exogenous HA on the cornea has been shown both in experimental animals (Miller, et al.: Use Of Na-Hyaluronate During Intraocular Lens Implantation In Rabbits. Ophthalmic Surgery, 8, (6), 58-61, 1977; Miller, et al.: Use Of Na-Hyaluronate In Autocomeal Transplantation In Rabbits. Ophthalmic Surgery, 11, (1), 19-21, 1980; Graue, et al.: The protective effect of Na-hyaluronate to comeal endothelium. Exp. Eye Res., 31, 119-127, 1980; Ozaki, et al.: Protective Effect Of Healon-Coated Intraocular Lens On The Corneal Endotheliurn. Folia Ophthalmologica Japonica, 32, 1301-1305, 1981) and In man (Norm.: Preoperative Protection Of Cornea And Conjunctiva. Acta Ophthalmologica, 59, 587-594, 1981; Polack F.M.et al.: Sodium hyaluronate (Healon) in keratoplasty and IOL implantation. Ophthalmology, 88, 425-431, 1981). During eye surgery, hyaluronic acid provides effective protection to exposed tissues, such as the corneal endothelium, and permits the reconstruction of the anatomical form of the operation site.

Exogenous hyaluronic acid introduced in the anterior or posterior chamber of the eye does not exert any negative effect on post surgical intraocular pressure, nor does it trigger any inflammatory sequelae in the intraocular environment. In addition, as opposed to other viscoelastic products, hyaluronic acid may be left in the eye as it is rapidly eliminated by physiological mechanisms. This property is very useful, especially during perforating keratoplasty or other eye lesions, where the removal of the injected substance is technically impracticable.

However, hyaluronic acid will typically decompose, or otherwise become altered, during extended storage periods, particularly at higher concentrations. One effect of the decomposition of HA is a marked reduction in molecular weight. The molecular weight of a particular fraction of HA is of special clinical importance in the uses contemplated. The biological activity of HA solutions depends generally on a combination of the molecular weight and conformation of the HA molecules and the concentration of these molecules in solution. There is an inverse relationship between HA molecular weight and concentration, such that higher concentrations of smaller HA molecules are required to achieve a given level of biological activity. Furthermore, reduction in molecular weight leads to a lower viscosity of the composition, eventually to a point at which the composition is no longer deemed sufficiently effective for such uses as ophthalmic surgery.

In order to have therapeutic activity, the concentration of HA in a therapeutically active solution should be at least the same magnitude as that which is found in normal tissue fluids, namely 0.1 - 0.3%. However, it is preferable that the concentration of HA in the therapeutic solution be higher than in normal tissue fluids, i.e., greater than about 0.3%, more preferably greater than about 0.5%, and most preferably greater than about 0.8%. The preferred composition according to the invention is a HA molecular weight of at least about 750,000, preferably at least about 1,200,000 and a concentration greater than about 1%; more preferably greater than about 1.5%

The composition described herein is buffered. The buffer maintains the pH preferably in the desired range, for example, in a physiologically acceptable range of about 4 or about 5 or about 6 to about 8 or about 9 or about 10. In particular, the solution preferably has a pH in the range of about 5.5 to about 8. The buffer is selected from inorganic or organic bases, preferably basic acetates, phosphates, borates, citrates, nitrates, sulfates, tartrates, lactates, carbonates, bicarbonates and mixtures thereof, more preferably basic phosphates, borates, citrates, tartrates, carbonates, bicarbonates and mixtures thereof. Typically, it is present in an amount of 0.001% to 2%, preferably 0.01% to 1%; most preferably from about 0.05% to about 0.30%.

The buffer component preferably includes one or more phosphate buffers, for example, combinations of monobasic phosphates, dibasic phosphates, and the like. Particularly useful phosphate buffers are those selected from phosphate salts of alkali and/or alkaline earth metals. Examples of suitable phosphate buffers include one or more of sodium dibasic phosphate (Na₂HPO₄), sodium monobasic phosphate (NaH₂PO₄), and potassium monobasic phosphate (KH₂PO₄).

The solutions described herein preferably include an effective amount of a tonicity component to provide the liquid medium with the desired tonicity. Such tonicity components may be present in the solution and/or may be introduced into the solution. Among the suitable tonicity adjusting components that may be employed are those conventionally used in contact lens care products, such as various inorganic salts. Sodium chloride and/or potassium chloride and the like are very useful tonicity components. The amount of tonicity component included is effective to provide the desired degree of tonicity to the solution. Such amount may, for example, be In the range of about 0.4% to about 1.5% (w/v). If a combination of sodium chloride and potassium chloride is employed, it is preferred that the weight ratio of sodium chloride to potassium chloride be in the range of about 3 to about 6 or about 8. The preferred tonicity component is sodium chloride present in the range of 0.50% to 0.90%.

Typical tonicity builders for use in the invention include suitable water soluble salts compatible with ocular tissue, preferably alkali or alkali earth metal halide, sulfates, nitrates, carbonates, borates, and phosphates, more preferably sodium or potassium chloride. The tonicity builder is present in an amount sufficient to provide a tonicity of the dosage regimen of 50 to 400 mosmol/kg, most preferably 250 to 350 mosmol/kg.

Thus, in a particularly preferred embodiment, the ophthalmic solution is a buffered saline solution comprising:
(a) greater than about 1 weight percent of hyaluronic acid; and
(b) about 0.0001 to 0.1 weight percent of a chelating agent having at least one phosphonic acid group.

The present method of stabilizing a buffered poly (carboxylic acid) solution at a concentration of 0.3 or higher, generally includes providing a buffered ophthalmically compatible composition including a poly (carboxylic acid); adding at least one strong, stable chelating agent, preferably including at least one phosphonic acid group, to the solution; and allowing the chelating agent to complex with the free metal ions present in the solution, which free metal ions may degrade the poly (carboxylic acid), i.e., "catalytic metal ions". This method is believed to allow for the formation of a metal ion complex and poly (carboxylic acid) formulation that has a decomposition rate that is less than the decomposition rate of the solution containing trace amounts of free catalytic metal ions.

The order of mixing the components is not believed to be critical. Thus, each of the components of the ophthalmic solution may be, separately and serially, added to a vessel containing water, or all the components may be added simultaneously. Preferably, the components are added separately, with dispersion or dissolution of each separate component being achieved prior to addition of the next component. However, the present stabilization method is not limited by the order of addition or contact of the components. Because the solutions of the present invention are contemplated to be used, *Inter alia,* in ophthalmic surgery, it is important in such cases, that the compositions be non-irritating to the internal environment of the eye. Thus, it is preferred that the compositions of the present invention be substantially free of hydrogen peroxide or compounds that generate hydrogen peroxide, such as sodium perborate.

Furthermore, the composition of the present invention may include a pharmaceutically active agent. For clarity of presentation, and not by way of limitation, the pharmaceutically active agents suitable for use in the present invention are divided into the following sections: (1) miotic agents; (2) mydriatic agents; and (3) anesthetic agents.

Suitable miotic agents include, but are not limited to, pilocarpine, isopilocarpine, pilocarpine hydrochloride, pilocarpine nitrate, isopilocarpine hydrochloride, isopilocarpine nitrate, carbachol, physostigmine, physostigmine sulfate, physostigmine sulfite, demecarium bromide, ecothiophate iodide and acetylcholine chloride. Preferred miotic agents are members of the pilocarpine and isopilocarpine family of compounds.

Suitable mydriatic agents include, but are not limited to, atropine, atropine sulfate, atropine hydrochloride, atropine methylbromide, atropine methylnitrate, atropine hyperduric, atropine N-oxide, phenylephrine, phenylephrine hydrochloride, hydroxyamphetamine, hydroxyamphetamine hydrobromide, hydroxy-amphetamine hydrochloride, hydroxyamphetamine iodide, cyclopentolate, cyclopentolate hydrochloride, homatropine, homatropine hydrobromide, homatropine hydrochloride, homatropine methylbromide, scopolamine, scopolamine hydrobromide, scopolamine hydrochloride, scopolamine methylbromide, scopolamine methyinitrate, scopolamine N-oxide, tropicamide, tropicamide hydrobromide, and tropicamide hydrochloride. Preferred mydriatic agents are members of the atropine family and phenylephrine family of compounds.

Suitable anesthetic agents include those that are cationic in charge (cationic amine salts) or potentially cationic in charge (uncharged amino groups), such agents comprising lidocaine, proparacaine, tetracaine, phenacaine, naepaine, lidocaine, cocaine, betoxycaine, bupivacaine, butacaine, butanilicalne, butoxycaine, carticaine, cyclomethycaine, dibucaine, dimethocaine, etidocaine, formcaine, hexylcaine, hydroxytetracaine, leucinocaine, mepivacaine, meprylcaine, metabutoxycaine, myrtecaine, octacaine, orthocaine, oxethazine, parethoxycaine, piperocaine, piridocaine, pfilocaine, procaine, propanocaine, propipocaine, propoxycaine, pseudocaine, pyrrocaine, ropivacaine, tolylcaine, tricaine and trimecaine. Preferred anesthetic agents are lidocaine, proparacane and tetracaine. The anesthetic agents of the invention may be used in their neutral, uncharged form or their charged, cationic form.

While the ideal concentration of the pharmaceutically active agent will depend on a number of factors, the concentration will generally fall within 0.001 and 10 weight percent. Preferably, the pharmaceutically active agent is present in an amount from about 0.01 to 2.0 weight percent. More preferably, the concentration of pharmaceutically active agent is about 0.1 to 1.5 weight percent. The preferred pharmaceutically active agent is an anesthetic; most preferably, lidocaine.

The previous disclosure will enable one having ordinary skill in the art to practice the invention. In order to better enable the reader to understand specific embodiments and the advantages thereof, reference to the following non-limiting examples is suggested.

### Example 1

A solution was prepared by adding BS5111 sodium hyaluronate (Fermentech Medical Limited, Lot #4916) to purified water in amounts sufficient to produce a 1 % sodium hyaluronate solution. The pH of the solution was 7.384.

### Example 2

A solution was prepared in the same manner as in Example 1, but with 180 ppm of DEQUEST 2060. The pH of the solution was 7.451.

### Example 3

A solution was prepared by adding BS5111 sodium hyaluronate to purified water in amounts sufficient to produce a 0.8% sodium hyaluronate solution. The pH of the solution was 7.190.

### Example 4

A solution was prepared in the same manner as in Example 3, but with 120 ppm of DEQUEST 2060. The pH of the solution was 7.289.

### Example 5

For all solutions, the viscosity of the sodium hyaluronate solution was measured initially and after exposure to a temperature of about 100 °0 for about 4 hours to calculate a relative recovery of sodium hyaluronate after heating. An elevated temperature is used in order to accelerate the stability testing. The results are shown in Table 1 below and demonstrate the effectiveness of the DEQUEST in stabilizing the sodium hyaluronate.

| Sample (with/without DEQUEST) | Viscosity before heated (cps) | Viscosity after heated (cps) | The viscosity recovery (%) |
|---|---|---|---|
| Example 1 (without) | 29180 | 5222, 5376 | 18.2 |
| Example 2 (with) | 26420 | 9676 | 36.6 |
| Example 3 (without) | 14590 | 1075 | 7.36 |
| Example 4 (with) | 11830, 11980 | 4608 | 38.7 |

## Claims

1. A stabilized solution, comprising:
a. greater than about 0.3 weight percent of at least hyaluronic acid; and
b. at least one strong and stable chelating agent having at least one phosphonic acid group in an amount of from 0.0001 to 0.1 weight percent; and
c. water.

2. A composition of claim 1, wherein said composition is ophthalmically compatible.

3. A composition of any one of the preceding claims, wherein said solution is substantially free of hydrogen peroxide and sources of hydrogen peroxide.

4. A composition of any one of the preceding claims, wherein the concentration of said hyaluronic acid is at least 0.8%.

5. A composition of any one of the preceding claims, wherein the concentration of said hyaluronic acid is at least 1 %.

6. A composition of any one of claims 1 to 5, wherein said chelating agent is selected from the group consisting of diethylene triamine penta(methylene phosphonic acid); hexamethylenediaminetetra (methylenephosphonic acid); ethylenediaminetetra (methylenephosphonic acid); aminotrimethylene phosphonates; and mixtures thereof.

7. A composition of claim 6, wherein said chelating agent is diethylene triamine penta(methylene phosphonic acid).

8. A composition of any one of the preceding claims, further comprising up to about 2 weight percent of a buffer.

9. A composition of any one of the preceding claims, further comprising 0.6 to 1.2 weight percent of a tonicity enhancer.

10. A composition of any one of claims 1 to 9, wherein said hyaluronic acid has an average molecular weight of at least 750,000.

11. A composition of claim 10, wherein said hyaluronic acid has an average molecular weight of at least 1,200,000.

12. A composition of any one of the preceding claims, further comprising a pharmaceutically active agent.

13. A composition of claim 12, wherein said pharmaceutically active agent is selected from the group consisting of miotic agents; mydriatic agents; and anaesthetic agents.

14. A composition of claim 13, wherein said pharmaceutically active agent is a miotic agent selected from the group consisting of pilocarpine, isopilocarpine, pilocarpine hydrochloride, pilocarpine nitrate, isopilocarpine hydrochloride, isopilocarpine nitrate, carbachol, physostigmine, physostigmine sulfate, physostigmine sulfite, demecarium bromide, ecothiophate iodide and acetylcholine chloride.

15. A composition of claim 13 or 14, wherein said miotic agent is selected from the group consisting of members of the pilocarpine and isopilocarpine family of compounds.

16. A composition of claim 13, wherein said pharmaceutically active agent is a mydriatic agent selected from the group consisting of atropine, atropine sulfate, atropine hydrochloride, atropine methylbromide, atropine methylnitrate, atropine hyperduric, atropine N-oxide, phenylephrine, phenylephrine hydrochloride, hydroxyamphetamine, hydroxyamphetamine hydrobromide, hydroxy-amphetamine hydrochloride, hydroxyamphetamine iodide, cyclopentolate, cyclopentolate hydrochloride, homatropine, homatropine hydrobromide, homatropine hydrochloride, homatropine methylbromide, scopolamine, scopolamine hydrobromide, scopolamine hydrochloride, scopolamine methylbromide, scopolamine methylnitrate, scopolamine N-oxide, tropicamide, tropicamide hydrobromide, and tropicamide hydrochloride.

17. A composition of claim 16, wherein said mydriatic agent is selected from the group consisting of members of the atropine family and phenylephrine family of compounds.

18. A composition of claim 13, wherein said pharmaceutically active agent is an anaesthetic agent selected from the group consisting of lidocaine, proparacaine, tetracaine, phenacaine, naepaine, cocaine, betoxycaine, bupivacaine, butacaine, butanilicaine, butoxycaine, carticaine, cyclomethycaine, dibucaine, dimethocaine, etidocaine, formcaine, hexylcaine, hydroxytetracaine, leucinocaine, mepivacaine, meprylcaine, metabutoxycaine, myrtecaine, octacaine, orthocaine, oxethazine, parethoxycaine, piperocaine, piridocaine, pfilocaine, procaine, propanocaine, propipocaine, propoxycaine, pseudocaine, pyrrocaine, ropivacaine, tolylcaine, tricaine and trimecaine.

19. A composition of claim 18, wherein said anaesthetic agent is selected from the group consisting of lidocaine, proparacaine and tetracaine.

20. A method of stabilizing a hyaluronic acid composition, comprising the step of preparing said hyaluronic acid composition including about 1 to 10 weight percent of hyaluronic acid or salts or mixture thereof and at least one strong and stable amine tri(lower alkylene phosphonic acid) chelating agent in an amount of from 0.0001 to 0.1 weight percent, wherein said chelating agent is capable of complexing with free catalytic metal ions to produce a composition with metal ion complexes and thereby reducing the decomposition rate of the hyaluronic acid in said composition.

21. A method of claim 20, wherein said chelating agent is selected from the group consisting of diethylene triamine penta(methylene phosphonic acid); hexamethylenediaminetetra (methylenephosphonic acid); ethylenediaminetetra (methylenephosphonic acid); aminotrimethylene phosphonates; and mixtures thereof.

22. A method of claim 21, wherein said chelating agent is diethylene triamine penta(methylene phosphonic acid).

## Patentansprüche

1. Stabilisierte Lösung, die Folgendes beinhaltet:
a. mehr als etwa 0,3 Gew.-% von wenigstens Hyaluronsäure; und
b. wenigstens einen starken und stabilen Chelatbildner mit wenigstens einer Phosphonsäuregruppe in einer Menge von 0,0001 bis 0,1 Gew.-%; und
c. Wasser.

2. Zusammensetzung nach Anspruch 1, wobei die genannte Zusammensetzung ophthalmisch kompatibel ist.

3. Zusammensetzung nach einem der vorherigen Ansprüche, wobei die genannte Lösung im Wesentlichen frei von Wasserstoffperoxid und Wasserstoffperoxidquellen ist.

4. Zusammensetzung nach einem der vorherigen Ansprüche, wobei die Konzentration der genannten Hyaluronsäure wenigstens 0,8% beträgt.

5. Zusammensetzung nach einem der vorherigen Ansprüche, wobei die Konzentration der genannten Hyaluronsäure wenigstens 1% beträgt.

6. Zusammensetzung nach einem der Ansprüche 1 bis 5, wobei der genannte Chelatbildner ausgewählt ist aus der Gruppe bestehend aus Diethylentriamin-penta(methylenphosphonsäure); Hexamethylendiamin-tetra(methylenphosphonsäure); Ethylendiamin-tetra(methylenphosphonsäure); Aminotrimethylenphosphonaten und Gemischen davon.

7. Zusammensetzung nach Anspruch 6, wobei der genannte Chelatbildner Diethylentriamin-penta(methylenphosphonsäure) ist.

8. Zusammensetzung nach einem der vorherigen Ansprüche, die ferner bis zu etwa 2 Gew.-% eines Puffers enthält.

9. Zusammensetzung nach einem der vorherigen Ansprüche, die ferner 0,6 bis 1,2 Gew.-% eines Tonizitätsverbesserers enthält.

10. Zusammensetzung nach einem der Ansprüche 1 bis 9, wobei die genannte Hyaluronsäure eine durchschnittliche Molekülmasse von wenigstens 750.000 hat.

11. Zusammensetzung nach Anspruch 10, wobei die genannte Hyaluronsäure eine durchschnittliche Molekülmasse von wenigstens 1.200.000 hat.

12. Zusammensetzung nach einem der vorherigen Ansprüche, die ferner ein pharmazeutisch aktives Mittel umfasst.

13. Zusammensetzung nach Anspruch 12, wobei das genannte pharmazeutisch aktive Mittel ausgewählt ist aus der Gruppe bestehend aus Miotika; Mydriatika und Anästhetika.

14. Zusammensetzung nach Anspruch 13, wobei das genannte pharmazeutisch aktive Mittel ein Miotikum ist, ausgewählt aus der Gruppe bestehend aus Pilocarpin, Isopilocarpin, Pilocarpinhydrochlorid, Pilocarpinnitrat, Isopilocarpinhydrochlorid, Isopilocarpinnitrat, Carbachol, Physostigmin, Physostigminsulfat, Physostigminsulfit, Demecariumbromid, Ecothiophatiodid und Acetylcholinchlorid.

15. Zusammensetzung nach Anspruch 13 oder 14, wobei das genannte Miotikum ausgewählt ist aus der Gruppe bestehend aus Mitgliedern der Pilocarpin- und der Isopilocarpin-Familie von Verbindungen.

16. Zusammensetzung nach Anspruch 13, wobei das genannte pharmazeutisch aktive Mittel ein Mydriatikum ist, das ausgewählt ist aus der Gruppe bestehend aus Atropin, Atropinsulfat, Atropinhydrochlorid, Atropinmethylbromid, Atropinmethylnitrat, Atropinhyperduric, Atropin-N-Oxid, Phenylephrin,
Phenylephrinhydrochlorid, Hydroxyamphetamin, Hydroxamphetaminhydrobromid, Hydroxyamphetaminhydrochlorid, Hydroxyamphetaminiodid, Cyclopentolat, Cyclopentolathydrochlorid, Homatropin, Homatropinhydrobromid, Homatropinhydrochlorid, Homatropinmethylbromid, Scopolamin, Scopolaminhydrobromid, Scopolaminhydrochlorid, Scopolaminmethylbromid, Scopolaminmethylnitrat, Scopolamin-N-Oxid, Tropicamid, Tropicamidhydrobromid und Tropicamidhydrochlorid.

17. Zusammensetzung nach Anspruch 16, wobei das genannte Mydriatikum ausgewählt ist aus der Gruppe bestehend aus Mitgliedern der Atropinfamilie und der Phenylephrinfamilie von Verbindungen.

18. Zusammensetzung nach Anspruch 13, wobei das genannte pharmazeutisch aktive Mittel ein Anästhetikum ist, das ausgewählt ist aus der Gruppe bestehend aus Lidocain, Proparacain, Tetracain, Phenacain, Naepain, Cocain, Betoxycain, Bupivacain, Butacain, Butanilicain, Butoxycain, Carticain, Cyclomethycain, Dibucain, Dimethocain, Etidocain, Formcain, Hexylcain, Hydroxytetracain, Leucinocain, Mepivacain, Meprylcain, Metabutoxycain, Myrtecain, Octacain, Orthocain, Oxethazin, Parethoxycain, Piperocain, Piridocain, Pfilocain, Procain, Propanocain, Propipocain, Propoxycain, Pseudocain, Pyrrocain, Ropivacain, Tolylcain, Tricain und Trimecain.

19. Zusammensetzung nach Anspruch 18, wobei das genannte Anästhetikum ausgewählt ist aus der Gruppe bestehend aus Lidocain, Proparacain und Tetracain.

20. Verfahren zum Stabilisieren einer Hyaluronsäurezusammensetzung, das den Schritt des Herstellens der genannten Hyaluronsäurezusammensetzung mit etwa 1 bis 10 Gew.-% Hyaluronsäure oder Salzen oder einem Gemisch davon und wenigstens einem starken und stabilen Amin-tri(niederalkylen-Phosphonsäure)-Chelatbildner in einer Menge von 0,0001 bis 0,1 Gew.-% beinhaltet, wobei sich der genannte Chelatbildner mit freien katalytischen Metallionen komplexieren kann, um eine Zusammensetzung mit Metallionenkomplexen zu erzeugen und dadurch die Zersetzungsrate der Hyaluronsäure in der genannten Zusammensetzung zu reduzieren.

21. Verfahren nach Anspruch 20, wobei der genannte Chelatbildner ausgewählt ist aus der Gruppe bestehend aus Diethylentriamin-penta(methylenphosphonsäure); Hexamethylendiamin-tetra(methylenphosphonsäure); Ethylendiamin-tetra(methylenphosphonsäure); Aminotrimethylenphosphonaten und Gemischen davon.

22. Verfahren nach Anspruch 21, wobei der genannte Chelatbildner Diethylentriamin-penta(methylenphosphonsäure) ist.

## Revendications

1. Solution stabilisée comprenant :
a. au moins l'acide hyaluronique à plus de 0,3 pour cent en poids environ ; et
b. au moins un agent chélateur puissant et stable ayant au moins un groupement acide phosphonique à une teneur comprise entre 0,0001 et 0,1 pour cent en poids ; et
c. de l'eau.

2. Composition de la revendication 1, où ladite composition est compatible sur le plan ophtalmique.

3. Composition de l'une quelconque des revendications précédentes, où ladite solution est essentiellement exempte de peroxyde d'hydrogène et de sources de peroxyde d'hydrogène.

4. Composition de l'une quelconque des revendications précédentes, où la concentration en ledit acide hyaluronique est de 0,8 % au moins.

5. Composition de l'une quelconque des revendications précédentes, où la concentration en ledit acide hyaluronique est de 1 % au moins.

6. Composition de l'une quelconque des revendications 1 à 5, où ledit agent chélateur est sélectionné dans le groupe consistant en l'acide diéthylènetriamine-penta(méthylène-phosphonique) ; l'acide hexaméthylènediamine-tétra(méthylène-phosphonique) ; l'acide éthylènediamine-tétra(méthylène-phosphonique) ; des phosphonates d'aminotriméthylène ; et des mélanges de ceux-ci.

7. Composition de la revendication 6, où ledit agent chélateur est l'acide diéthylènetriamine-penta(méthylène-phosphonique).

8. Composition de l'une quelconque des revendications précédentes, qui comprend également un tampon à jusqu'à 2 pour cent en poids environ.

9. Composition de l'une quelconque des revendications précédentes, qui comprend également un agent augmentant la tonicité à 0,6 à 1,2 pour cent en poids.

10. Composition de l'une quelconque des revendications 1 à 9, où ledit acide hyaluronique a un poids moléculaire moyen de 750 000 au moins.

11. Composition de la revendication 10, où ledit acide hyaluronique a un poids moléculaire moyen de 1 200 000 au moins.

12. Composition de l'une quelconque des revendications précédentes, qui comprend également un agent pharmaceutiquement actif.

13. Composition de la revendication 12, où ledit agent pharmaceutiquement actif est sélectionné dans le groupe consistant en des agents myotiques, des agents mydriatiques et des agents anesthésiques.

14. Composition de la revendication 13, où ledit agent pharmaceutiquement actif est un agent myotique sélectionné dans le groupe consistant en les suivants : pilocarpine, isopilocarpine, chlorhydrate de pilocarpine, nitrate de pilocarpine, chlorhydrate d'isopilocarpine, nitrate d'isopilocarpine, carbachol, physostigmine, sulfate de physostigmine, sulfite de physostigmine, bromure de démécarium, iodure d'écothiophate et chlorure d'acétylcholine.

15. Composition de la revendication 13 ou 14, où ledit agent myotique est sélectionné dans le groupe consistant en des composés de la famille de la pilocarpine et de l'isopilocarpine.

16. Composition de la revendication 13, où ledit agent pharmaceutiquement actif est un agent mydriatique sélectionné dans le groupe consistant en les suivants : atropine, sulfate d'atropine, chlorhydrate d'atropine, méthylbromure d'atropine, méthylnitrate d'atropine, atropine à action prolongée, N-oxyde d'atropine, phényléphrine, chlorhydrate de phényléphrine, hydroxyamphétamine, hydrobromure d'hydroxyamphétamine, chlorhydrate d'hydroxyamphétamine, iodure d'hydroxyamphétamine, cyclopentolate, chlorhydrate de cyclopentolate, homatropine, hydrobromure d'homatropine, chlorhydrate d'homatropine, méthylbromure d'homatropine, scopolamine, hydrobromure de scopolamine, chlorhydrate de scopolamine, méthylbromure de scopolamine, méthylnitrate de scopolamine, N-oxyde de scopolamine, tropicamide, hydrobromure de tropicamide et chlorhydrate de tropicamide.

17. Composition de la revendication 16, où ledit agent mydriatique est sélectionné dans le groupe consistant en des composés de la famille de l'atropine et de la phényléphrine.

18. Composition de la revendication 13, où ledit agent pharmaceutiquement actif est un agent anesthésique sélectionné dans le groupe consistant en les suivants : lidocaïne, proparacaïne, tétracaïne, phénacaïne, naépaïne, cocaïne, bétoxycaïne, bupivacaïne, butacaïne, butanilicaïne, butoxycaïne, carticaïne, cyclométhycaïne, dibucaïne, diméthocaïne, étidocaïne, formcaïne, hexylcaïne, hydroxytétracaïne, leucinocaïne, mépivacaïne, méprylcaïne, métabutoxycaïne, myrtécaïne, octacaïne, orthocaïne, oxethazine, paréthoxycaïne, pipérocaïne, piridocaïne, pfilocaïne, procaïne, propanocaïne, propipocaïne, propoxycaïne, pseudocaïne, pyrrocaïne, ropivacaïne, tolylcaïne, tricaïne et trimécaïne.

19. Composition de la revendication 18, où ledit agent anesthésique est sélectionné dans le groupe consistant en la lidocaïne, la proparacaïne et la tétracaïne.

20. Méthode de stabilisation d'une composition à base d'acide hyaluronique, comprenant l'étape de préparation d'une composition à base d'acide hyaluronique qui contient de 1 à 10 pour cent en poids environ d'acide hyaluronique ou de ses sels ou d'un mélange de ceux-ci et au moins un agent chélateur puissant et stable qui est un acide amine-tri(alkylène inférieur-phosphonique) à une quantité comprise entre 0,0001 et 0,1 pour cent en poids, où ledit agent chélateur est capable de se complexer à des ions métalliques catalytiques libres pour produire une composition avec des complexes d'ions métalliques et réduire ainsi le taux de décomposition de l'acide hyaluronique dans ladite composition.

21. Méthode de la revendication 20, où ledit agent chélateur est sélectionné dans le groupe consistant en l'acide diéthylènetriamine-penta(méthylène-phosphonique) ; l'acide hexaméthylènediamine-tétra(méthylène-phosphonique) ; l'acide éthylènediamine-tétra(méthylène-phosphonique) ; des phosphonates d'aminotriméthylène ; et des mélanges de ceux-ci.

22. Méthode de la revendication 21, où ledit agent chélateur est l'acide diéthylènetriamine-penta(méthylène-phosphonique).
